# EUROPÄISCHE PATENTANMELDUNG

(11) **EP 1 889 574 A2**
(43) Veröffentlichungstag der Anmeldung: **20.02.2008**
(21) Anmeldenummer: 07014543.8
(22) Anmeldetag: 24.07.2007
(51) Int. Cl.: A61B 17/00

(54) **Klammer zur Befestigung chirurgischer Operationshilfsmittel, Stellorgan für eine derartige Klammer und eine Anordnung umfassend ein Stellorgan und eine Klammer**

(30) Priorität: 31.07.2006 DE 102006035602
(71) Anmelder: Plus Orthopedics AG, 6343 Rotkreuz (CH)
(72) Erfinder: Hauri, Bernhard, 5053 Staffelbach (CH)
(74) Vertreter: Popp, Eugen

(57) **Zusammenfassung**

Die Erfindung betrifft eine Klammer zur Befestigung chirurgischer Operationshilfsmittel (14) mit mindestens zwei relativ zueinander beweglich angeordneten Klammerteilen (1, 2; 17, 19), mit einem Arretierelement (30) zur Festlegung der Klammerteile (1, 2; 17, 19) in mindestens einer Sperrstellung, wobei die Klammerteile (1, 2; 17, 19) Anschlussmittel (7', 7") aufweisen derart, dass die Klammerteile (1, 2; 17, 19) mit einem separaten Stellorgan (9) zur Übertragung einer Stellkraft verbindbar sind. Die Erfindung betrifft des weiteren ein Stellorgan (9), insbesondere eine Zange, zur lösbaren Verbindung mit einer Klammer (1, 2; 17, 19), das Anschlussmittel (10, 10a) aufweist, die mit den Anschlussmitteln (7', 7") der Klammer (1, 2; 17, 19) verbindbar sind und Abstands- und/oder Ausrichtmittel umfasst. Die Erfindung betrifft ferner eine Anordnung zur Befestigung chirurgischer Operationsmittel umfassend eine Klammer und ein mit der Klammer lösbar verbundenes Stellorgan (9).

## Beschreibung

Die Erfindung bezieht sich auf eine Klammer zur Befestigung chirurgischer Operationshilfsmittel, ein Stellorgan für eine derartige Klammer und eine Anordnung umfassend ein Stellorgan und eine Klammer.

Eine Klammer mit den Merkmalen des Oberbegriffs des Anspruchs 1 ist beispielsweise aus der WO 00/00093 bekannt.

Derartige Klammern werden zum temporären Fixieren von Operationshilfsmitteln an Knochen, insbesondere Röhrenknochen, verwendet, um bei chirurgischen Eingriffen, wie Einsetzen von Gelenkersatzimplantaten, eine feste Ortsstabilität zwischen dem Operationshilfsmittel und dem Knochen zu gewährleisten.

Chirurgische Eingriffe zur Ersetzung von Gelenken oder Gelenkbestandteilen bei Menschen sind seit langem bekannt und gehören zum klinischen Alltag. Parallel zur Weiterentwicklung der Implantatkonstruktionen werden auch geeignete Operationstechniken und -hilfsmittel bereitgestellt, insbesondere auch an die jeweiligen Implantatkonstruktionen angepasste Werkzeuge zum Einsetzen derselben.

In den letzten Jahren wurde eine größere Zahl neuer Positionierungshilfsmittel und - Verfahren entwickelt.

WO 03/096870 A2, die auf die Anmelderin zurückgeht, offenbart eine Anordnung und ein Verfahren zur intraoperativen Festlegung der Lage eines Gelenkersatzimplantats. Dabei wird mit Hilfe der Navigationstechnik während einer Operation laufend die Lage von Skelettteilen in einem dreidimensionalen Koordinationssystem erfasst. Ein Mehrkamerasystem erfasst dabei so genannte Mehrpunktgeber - in der Praxis oft auch Markerfeld oder Lokator genannt - welche ihrerseits ortsfest mit dem entsprechenden Skelettteil verbunden sind. Durch Antasten interessierender Stellen am Skelettteil mittels Messtaster werden die Antaststellen dem durch das Markerfeld repräsentierten Koordinationssystem zugeordnet. In Fig. 3 der WO 03/096870 A2 ist eine Femurklammer gezeigt, von der zwei Zapfen zum Aufstecken eines Lokators abstehen.

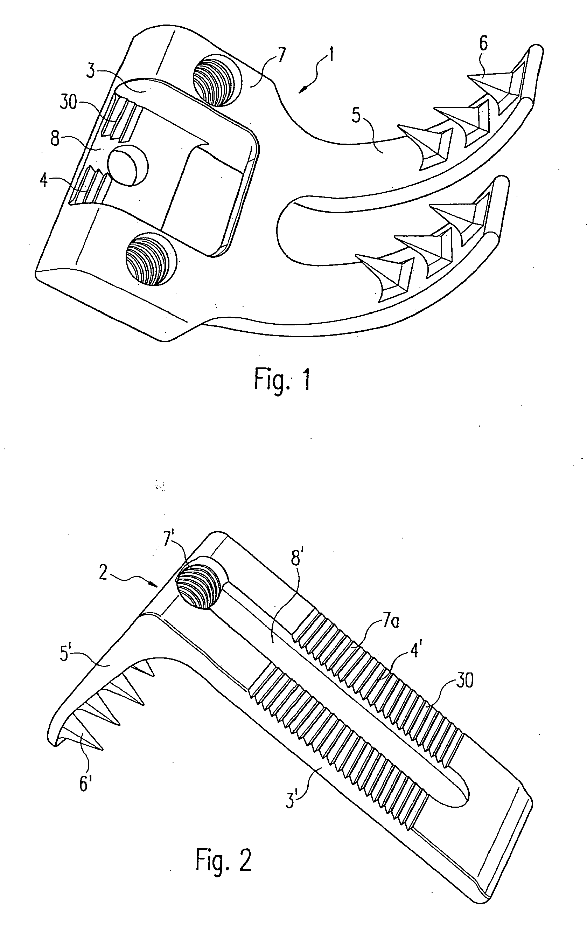

WO 00/00093 A1, die ebenfalls auf die Anmelderin zurückgeht, beschreibt ein umfangreiches Instrumentarium zum Einsetzen eines Kniegelenkimplantates. Die dabei offenbarten Positionshilfsmittel basieren sowohl auf Verschraubungstechnik der Hilfsmittel mit dem zu bearbeitenden Knochen (Fig. 1) als auch auf einer Klammertechnik (Fig. 16), wobei der die Klammerspannkraft gebende Teil in Form von Spindeltrieben einen integralen Teil der Klammer bilden. Die dergestalt mit dem Knochen verbundenen Operationshilfsmittel bilden die Basis einer per Schnellverschluss aufzubauenden Mehrachs-Minirobotereinheit, welche zugleich Mess- als auch Schneidelehrenfunktion für die Resektionsschnitte zu erfüllen hat.

Eine häufig verwendete Methode der Fixierung von Klammern ist ein Schließmechanismus angetrieben durch eine Gewindespindel, wie in der WO 00/00093 A1 offenbart. Diese Spindelantriebe erzeugen die Schließkraft der gegeneinander opponierend bewegbaren Klammerschenkel. Spindelantriebe haben allerdings den Nachteil eines relativ großen Bauvolumens. Nach dem Schließen der Klammer, d. h. während der eigentlichen Funktion der Klammer als Träger eines Operationshilfsmittels während der Operation, hat die in die Klammer integrierte Spindel nur noch Haltefunktion.

Vielen der bisher bekannten Einrichtungen zur temporären Befestigung von Operationshilfsmitteln an Skelettteilen im Zusammenhang mit Gelenkersatzoperationen weisen folgende Nachteile auf:
- Die Bauform und Größe führt zu einer umfangreichen invasiven Gestaltung im Operationsumfeld.
- Mechanische Kerben durch in den Knochen eingebrachte Bohrungen für Schrauben und Stifte erhöhen das Risiko von Frakturen.

Der Erfindung liegt die Aufgabe zugrunde, eine Klammer zur Befestigung chirurgischer Operationshilfsmittel anzugeben, die sicher mit dem jeweiligen Knochen verbunden werden kann, wobei die Beeinträchtigung des umgebenden Gewebes möglichst gering gehalten werden soll. Ferner soll ein Stellorgan für eine derartige Klammer sowie eine Anordnung umfassend ein Stellorgan und eine Klammer angegeben werden.

Erfindungsgemäß wird die Aufgabe durch eine Klammer mit den Merkmalen des Anspruchs 1, ein Stellorgan mit den Merkmalen des Anspruchs 8 sowie eine Anordnung mit den Merkmalen des Anspruchs 10 gelöst.

Die Erfindung beruht demnach auf dem Gedanken, eine Klammer zur Befestigung chirurgischer Operationshilfsmittel vorzusehen, die mindestens zwei relativ zueinander beweglich angeordnete Klammerteile sowie ein Arretierelement zur Festlegung der Klammerteile in mindestens einer Sperrstellung aufweist. Die Klammerteile weisen Anschlussmittel auf derart, dass die Klammerteile mit einem separaten Stellorgan zur Übertragung einer Stellkraft verbindbar sind, wobei vorzugsweise die Klammerteile mittels des Stellorgans in die Sperrstellung und/oder in eine Freigabestellung bringbar sind. Weiter vorzugsweise ist die Klammer mittels des Stellorgans setzbar, spannbar und wieder lösbar, wobei dazu das Arretierelement aus der Sperrstellung in eine Freigabestellung und umgekehrt bewegbar angeordnet ist.

Das bedeutet, dass der eigentliche Aufsetzvorgang der Klammer auf den Knochen über ein entfernbares Aufsetzhilfsmittel, d. h. das Stellorgan erfolgt, das die Stellkraft bzw. Aufspannkraft auf die Klammerteile überträgt. Nachdem die Klammer an der gewünschten Stelle angebracht ist, kann das Stellorgan entfernt werden. Dabei wird die Klammer durch das Arretierelement gesperrt und in der gewünschten Lage gehalten, wobei der jeweilige Knochen zumindest bereichsweise von der Klammer umschlossen ist. Die Klammer selbst muss daher nur die Arretierfunktion erfüllen, nicht aber, wie bisher im Stand der Technik, Mittel zum Verspannen der Klammerteile aufweisen (Spindeltrieb). Die Funktionen, die die Klammer nach dem Aufsetzen auf den Knochen für die weitere Dauer der Operation zu erfüllen hat, sind daher auf ein Minimum beschränkt. Dies hat zur Folge, dass die Größe und Komplexität der Klammer reduziert werden kann. Die reduzierte Größe und Bauform der erfindungsgemäßen Klammer ist insofern wichtig, als die Knochen, besonders im gelenknahen Bereich, von Muskel- und Sehnenzügen umgeben sind, die wichtige mechanische Funktionen haben und daher durch die Größe und Bauform der Klammer möglichst wenig irritiert werden sollten. Ein wichtiges Kriterium für eine erfolgreiche Implantation einer Gelenkendoprothese ist eine korrekte Spannung der gelenkstabilisierenden Weichteilstrukturen, die durch große Bauformen, insbesondere mit hervorstehenden Elementen, negativ beeinflusst werden, so dass die mechanischen Verhältnisse, wie zum Beispiel die Spannung der gelenküberbrückenden Weichteile verändert werden. Die erfindungsgemäß ermöglichte kompakte Bauform der Klammer führt dazu, dass vor und nach der Entfernung der Klammer im Wesentlichen die selben mechanischen Verhältnisse herrschen.

Die Verringerung des Bauvolumens wird maßgeblich dadurch erreicht, dass bei der erfindungsgemäßen Klammer ein externes, d. h. entfernbares Schließkraft gebendes Element bzw. allgemein Stellorgan an die Klammer angekoppelt und nach dem Setzen der Klammer entfernt werden kann. Die Haltefunktion der Klammer wird durch die Arretiervorrichtung erfüllt.

Die kompakte Bauform der erfindungsgemäßen Klammer hat ferner den Vorteil, dass im Fall von Implantaten mit schaftförmigen, im Röhrenknochen zu platzierenden Bauteilen Kollisionen zwischen den Befestigungselementen von Operationshilfsmitteln und den Instrumenten zur Vorbereitung der Schaftverankerung oder den Verankerungsschäften selbst vermieden werden.

Die Ankopplung bzw. Abkopplung des Stellorgans mit bzw. von der Klammer erfolgt über die an der Klammer vorgesehenen Anschlussmittel, die so ausgebildet sind, dass das Stellorgan mit diesen Anschlussmitteln lösbar verbunden werden kann.

Vorzugsweise sind die Anschlussmittel für das separate Stellorgan zur Befestigung der Operationshilfsmittel angepasst. Dies bedeutet, dass die Anschlussmittel bzw. wenigstens ein Anschlussmittel sowohl mit dem Stellorgan, als auch - nach Entfernen des Stellorgans - mit einem Operationshilfsmittel verbunden werden kann. Durch diese Funktionsüberlagerung wird das Bauvolumen der Klammer weiter reduziert. Die Anschlussmittel sowohl auf Seiten der Klammer als auch auf Seiten des Stellorgans sind einfach, bedien- und funktionssicher und insbesondere auf Seiten der Klammer nicht auftragend oder vorstehend ausgebildet. Das Anschlussmittel ist ferner so ausgebildet, dass bei mehrmaligem Ankoppeln eines Operationshilfsmittels dieses immer in die genau gleiche Relativlage zum Knochen gebracht wird.

Bei einer bevorzugten Ausführungsform sind die Klammerteile durch eine Linearführung oder durch ein Drehgelenk verbunden. Generell sind die Führungen der Klammerteile so ausgebildet, dass sich durch eine Relativbewegung der Klammerteile zur Einstellung der gewünschten Klemmdimension bzw. durch Ineinanderschieben eine Reaktionskraft des gespannten Knochens ergibt, die einen vor dem Erreichen der Klemmdimension bestehenden Kipp-Freiheitsgrad der Klammerteile relativ zueinander aufhebt. Die Linearführung bzw. das Drehgelenk bieten somit eine einfache Möglichkeit, die beiden Klammerteile in die zum Festklemmen des Knochens erforderliche Halteposition zu bringen.

Vorzugsweise weist das Arretierelement Rastmittel, insbesondere Zähne, auf, die den Klammerteilen zugeordnet und in der Sperrstellung verrastbar sind. Dadurch kann die Erhaltung der Aufsetzspannung bzw. Klemmspannung einfach erreicht werden.

Bei einer weiteren bevorzugten Ausführungsform ist ein Löseelement vorgesehen, dass mit dem Arretierelement zum Lösen der Klammerteile aus der Sperrstellung zusammenwirkt. Auf diese Weise kann die fixierte Klammer leicht vom Knochen gelöst werden.

Es ist auch möglich, das Arretierelement so anzuordnen, dass dieses aus der Sperrstellung in eine Freigabestellung und umgekehrt bewegbar ist. Auch auf diese Weise wird die Freigabe des Knochens ermöglicht.

Dabei kann das Arretierelement mit einer Rückstellkraft beaufschlagt sein, die das Arretierelement in die Sperrstellung bewegt und/oder in der Sperrstellung hält. Damit wird die Sicherheit der Klammerverbindung erhöht, so dass eine reproduzierbare Befestigung der Operationshilfsmittel in der gewünschten Lage gewährleistet ist.

Bei dem erfindungsgemäßen Stellorgan können Abstands- und/oder Ausrichtmittel vorgesehen sein, die eine Handhabung des Stellorgans vereinfachen.

Die Erfindung wird nachfolgend anhand von Ausführungsbeispielen unter Bezug auf die beigefügten Zeichnungen mit weiteren Einzelheiten beschrieben.

Darin zeigen
- Fig. 1: eine perspektivische Darstellung eines ersten Ausführungsbeispiels eines Klammerteils mit Innenführungskontur;
- Fig. 2: eine perspektivische Darstellung eines weiteren Klammerteils mit Außenführungskontur gemäß dem ersten Ausführungsbeispiel der Erfindung;
- Fig. 3a: eine Klammer umfassend die Klammerteile gemäß den Figuren 1, 2, wobei die Klammer auf einen Knochen aufgespannt ist;
- Fig. 3b: die Klammer gemäß Fig. 3a ohne Knochen;
- Fig. 4: die Klammer gemäß Fig. 3a, an der Aufsetzhilfsmittel bzw. ein Stellorgan befestigt ist;
- Fig. 4a: die Klammer und das Stellorgan gemäß Fig. 4 in verschiedenen Stellungen;
- Fig. 5: die Klammer gemäß Fig. 3a mit einem angekoppelten Lokator;
- Fig. 6a: ein zweites Ausführungsbeispiel der erfindungsgemäßen Klammer mit eingekoppeltem Arretierbügel;
- Fig. 6b: die Klammer gemäß Fig. 6a mit ausgekoppeltem Arretierbügel; und
- Fig. 7: einen Schnitt durch die Anordnung aus Klammer und Aufsetzhilfsmittel bzw. Stellorgan gemäß Fig. 6a;
- Fig. 7a: eine Detailansicht der Zahneingriffspartie gemäß Fig. 7; und
- Fig. 7b: eine perspektivische Darstellung der Anordnung gemäß Fig. 7.

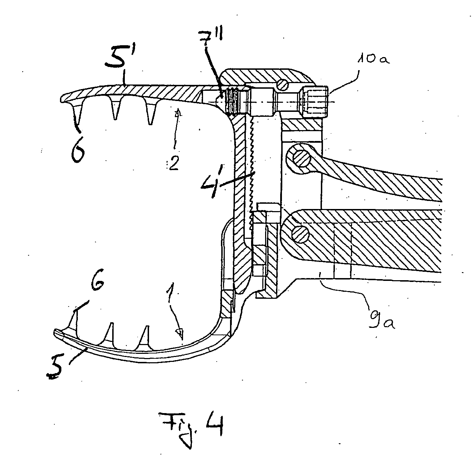

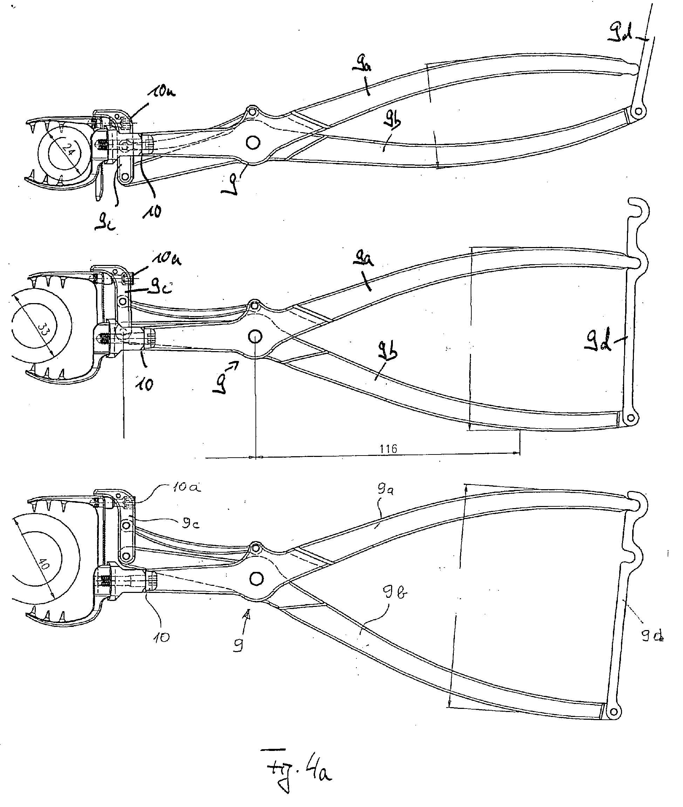

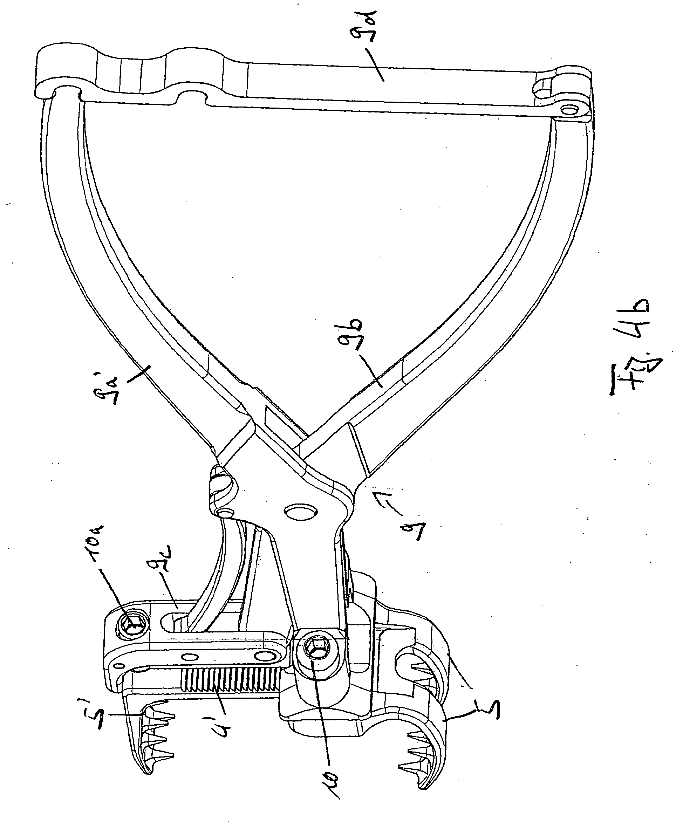

Gemäß den Figuren 1 bis 4 umfasst ein erstes Ausführungsbeispiel einer Knochenklammer die erste Klammerhälfte 1 bzw. allgemein das Klammerteil 2 in Fig. 1 sowie die zweite Klammerhälfte 2 bzw. allgemein das Klammerteil 2 in Fig. 2. Am Führungs-/Adaptionsteil 7 der ersten Klammerhälfte 1 sind in diesem Ausführungsbeispiel zwei Klammerarme 5 mit je daran ausgebildeten Verankerungselementen 6 vorgesehen. Dadurch entsteht eine stabile Verankerung der Klammer auf dem Knochen. Am Führungs-/Adaptionsteil 7 der anderen (zweiten) Klammerhälfte ist ein weiterer Klammerarm 5' vorgesehen, der im Gebrauch den beiden anderen Klammerarmen 5 gegenüber angeordnet ist.

Die Führung der beiden Klammerhälften 1, 2 ist als Linearführung mit einer Innenführung 3 der ersten Klammerhälfte 1 sowie einer Außenführung 3' der zweiten Klammerhälfte 2 realisiert. Ein dabei bestehender hipp-Freiheitsgrad wird in der ersten Phase des Zusammenschiebens durch die Parallelogrammführung des Aufsetzhilfsmittels 9 begrenzt, beziehungsweise nach Entstehen der beidseits wirkenden Spann-Reaktionskraft des Knochens auf die Klammerarme 5 bzw. 5' ohnehin eliminiert.

Als Formschlusselemente bzw. Rastelemente gegen das ungewollte Öffnen der aufgesetzten Klammer dient hier eine über eine größere Länge der zweiten Klammerhälfte 2 angeordnete Anzahl Zähne 4' mit Sägezahnform. Diese Sägezähne 4' kommen spätestens ab beginnender Knochenreaktionskraft auf die Klammer mit den nur über kurze Länge angeordneten Sägezähnen 4 der ersten Klammerhälfte 1 in gegenseitigen Eingriff. Durch diese Anordnung werden durch die Knochenreaktionskraft die Sägezähne 4 bzw. 4' gegen den Zahnlückengrund der Gegenverzahnung gedrückt und so die beiden Klammerhälften 1, 2 gegenseitig sicher in Formschluss gehalten. Durch unterschiedliche Gestaltung der Zahnwinkel können härter zu schließende Klammern oder weicher zu schließende Klammern erzielt werden. Ebenso wird durch unterschiedliche Zahnwinkel die Arretierungssicherheit gegen außerordentliche Krafteinwirkungen und Schwingungen verändert.

Anstelle der Zahnverbindung kann das Arretierelement auf andere Weise realisiert werden. Denkbar sind beispielsweise der Einsatz von Rastklinken oder anderen Formschlußelementen.

Anstelle der Linearführung können auch andere Verbindungen zwischen den beiden Klammerteilen 1, 2 vorgesehen sein, die eine Relativbewegung der beiden Klammerteile 1, 2 ermöglichen. Dies können beispielsweise Gelenkverbindungen, insbesondere Drehgelenke sein.

Die Anschlußmittel sind jeweils im Bereich der Adaptionspartien bzw. Adaptionsteilen 7, 7a der beiden Klammerteile 1, 2 bzw. Klammerhälften 1, 2 angeordnet. Die Anschlußmittel sind als Gewindebohrungen 7', 7" ausgeführt und zur Aufnahme passender Schrauben dimensioniert, die in dem separaten Stellorgan 9 bzw. Aufsetzhilfsmittel gelagert sind. Anstelle der Schraubverbindungen können auch andere Verbindungsarten gewählt werden, die eine lösbare Verbindung der beiden Klammerteile 1, 2 mit dem Stellorgan 9 zur Übertragung einer Stellkraft vom Stellorgan 9 auf die beiden Klammerteile 1, 2 ermöglichen. Dabei können beispielsweise kraft- bzw. formschlüssige Verbindungen zum Einsatz kommen.

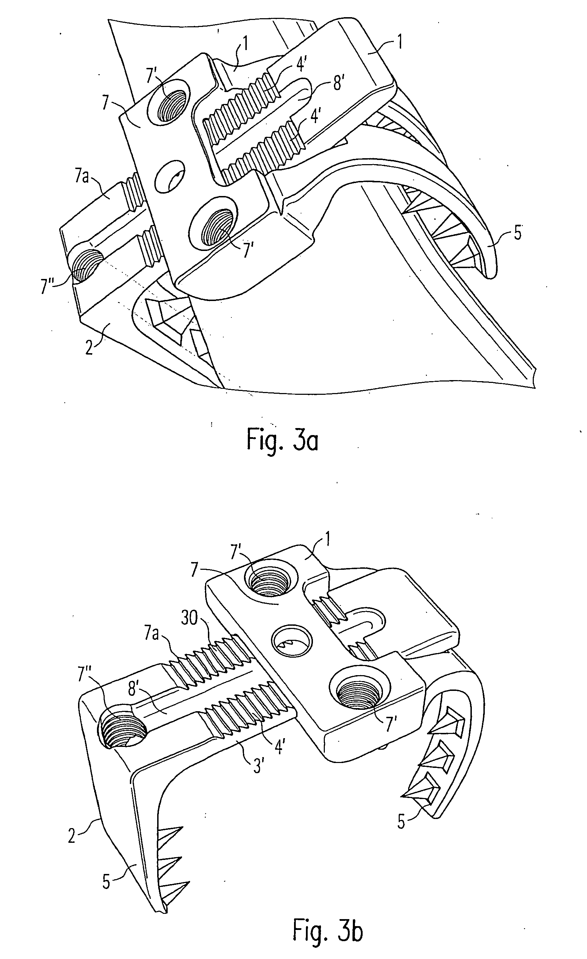

Bei dem in den Figuren 1 bis 4 dargestellten Ausführungsbeispiel sind drei Anschlußmittel, d.h. drei Gewindebohrungen 7', 7" vorgesehen. Zwei der Gewindebohrungen 7' sind im Adaptionsteil 7 des zweiten Klammerteiles 2 mit der Außenführung 3' vorgesehen. Die Adaptionspartien bzw. das Adaptionsteil 7 des ersten Klammerteiles 1 mit der Innenführung 3 erstreckt sich im wesentlichen quer zum Adaptionsteil 7a des anderen Klammerteiles 2, wie in den Fig. 3a, 3b zu erkennen. Zwei der Anschlußmittel 7' sind in dem sich quer erstreckenden Adaptionsteil 7 angeordnet, und zwar nahe den Außenkanten des A-daptionsteils 7. Das dritte Anschlußmittel 7" ist im Adaptionsteil 7a des anderen Klammerteiles 2 angeordnet, und zwar nahe der Kante, wo der Klammerarm 5 mit dem Adaptionsteil 7a verbunden ist. Das Anschlußmittel 7" des anderen (zweiten) Klammerteiles 2 ist ferner mittig zwischen den beiden anderen Anschlußmitteln 7' vorgesehen. Durch diese symmetrische Anordnung der Anschlußmittel 7', 7" wird ein Verkanten der beiden Klammerteile 1, 2 vermieden, wenn das Stellorgan 9 an den Anschlußmitteln 7', 7" angekoppelt ist und zur Übertragung der Stellkraft betätigt wird.

Andere Anordnungen der Anschlußmittel sind ebenfalls möglich.

Der Aufsetzvorgang der Knochenklammer auf den Knochen geschieht mit dem Aufsetzmittel 9 bzw. allgemein Stellorgan 9, welches zwei schergelagerte Armteile 9a, 9b aufweist. Vorbereitend werden die beiden Klammerhälften 1 und 2 am Aufsetzhilfsmittel 9 angebracht. Ein Armteil 9a besitzt auf der Frontseite eine zur Adaptionspartie 7 von Klammerhälfte 1 formschlüssig korrespondierende Adaptionsschnittstelle und eine Schraube 10, zur sicheren Verbindung der ersten Klammerhälfte 1 mit dem Aufsetzhilfsmittel 9. Am zweiten Armteil 9b befindet sich auf der Frontseite ein parallelogrammgeführter Winkelausleger 9c, welcher die zweite Klammerhälfte 2 außen umschließt. Zur Halterung mit gewissem Freiheitsgrad der zweiten Klammerhälfte 2 am Aufsetzhilfsmittel 9 dient eine Schraube 10a.

Operationsvorbereitend kann das Aufsetzhilfsmittel 9 mittels schwenkbarem Schnappbügel 9d in zwei Größenstellungen fixiert werden.

Bei Beginn des eigentlichen Aufsetzvorganges führt der Operateur das so vorbereitete Aufsetzhilfsmittel 9 an die gewünschte Knochenstelle heran, löst den Schnappbügel 9d und betätigt per Handkraft auf die Arme 9a, 9b den Schließvorgang der Knochenklammer. Sobald dabei eine Reaktionskraft des Knochens auf die Klammerarme 5 erfolgt, kommen die Zahnrücken der Zähne 4 mit denjenigen der Zähne 4' in Auflage. Ein Weiterführen des Schließvorganges hat nun zunehmenden Widerstand zur Folge und der Klammereingriff erfolgt zunehmend in mit größerer Spannkraft verbundenen Zahneingriffkombinationen, bis die gewünschte Spannkraft der Knochenklammer erreicht ist.

Zum Zweck des gewollten Lösens der Knochenklammer vom Knochen wird gegen die elastische Spannungssituation der beiden Klammerhälften 1, 2 eine Öffnungskraft zwischen die Öffnungsspur 8' und die Klammerpartie 8 mittels eines Kipphebelwerkzeugs angelegt, bis die im Eingriff befindlichen Zähne 4, 4' außer Eingriff kommen und sich somit die Klammerhälften 1, 2 voneinander lösen und vom Knochen entfernt werden können.

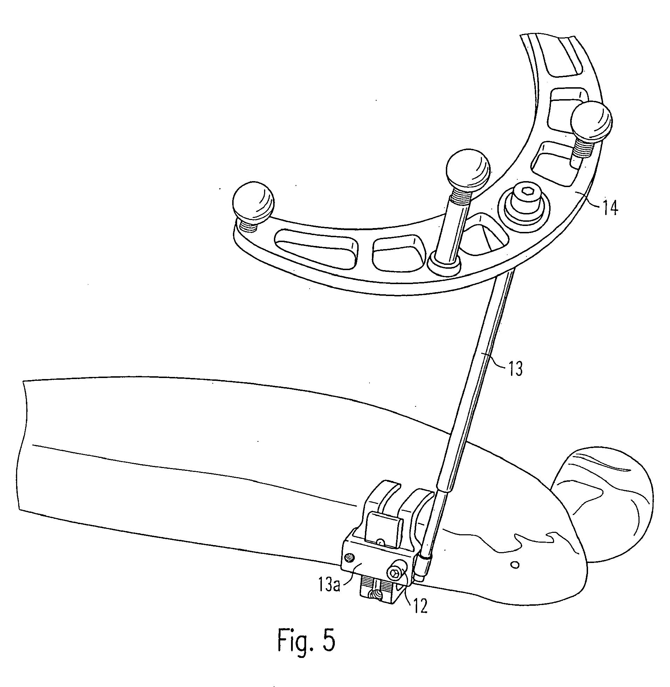

Fig. 5 zeigt ein Markerfeld 14 mit angekoppelter Tragstange 13, deren adapterseitige Endpartie 13a mittels eines Verbindungselements 12 an der auf einen Knochen fixierten Knochenklammer gemäß dem ersten Ausführungsbeispiel befestigt ist.

Die Erfindung ist nicht auf Klammern bestehend aus zwei Klammerteilen bzw. zwei Klammerhälften beschränkt, sondern kann auch auf Klammern mit mehr als zwei Klammerteilen angewendet werden, die zur Erzeugung einer Klemmkraft relativ zueinander, insbesondere opponierend bewegbar sind.

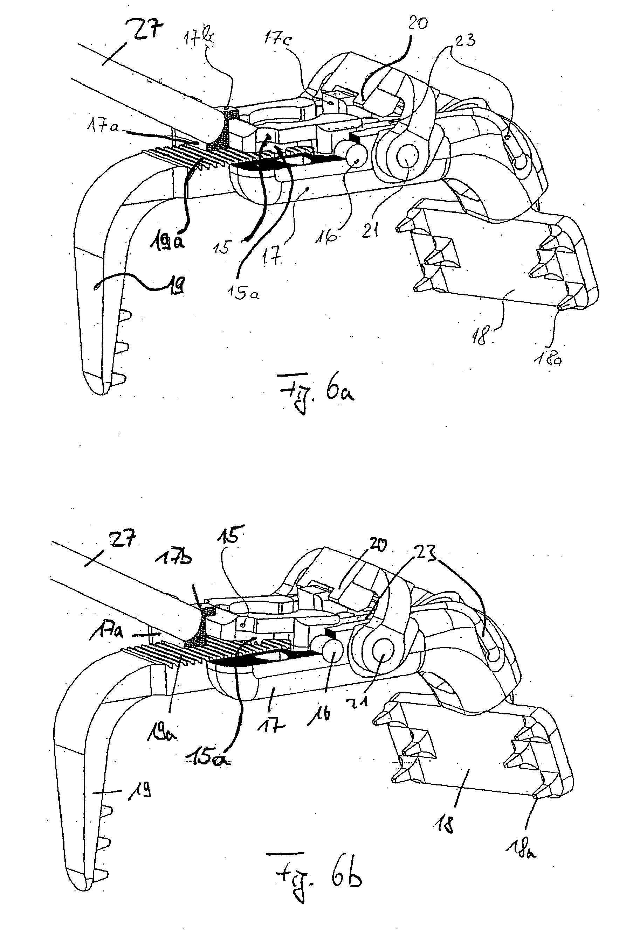

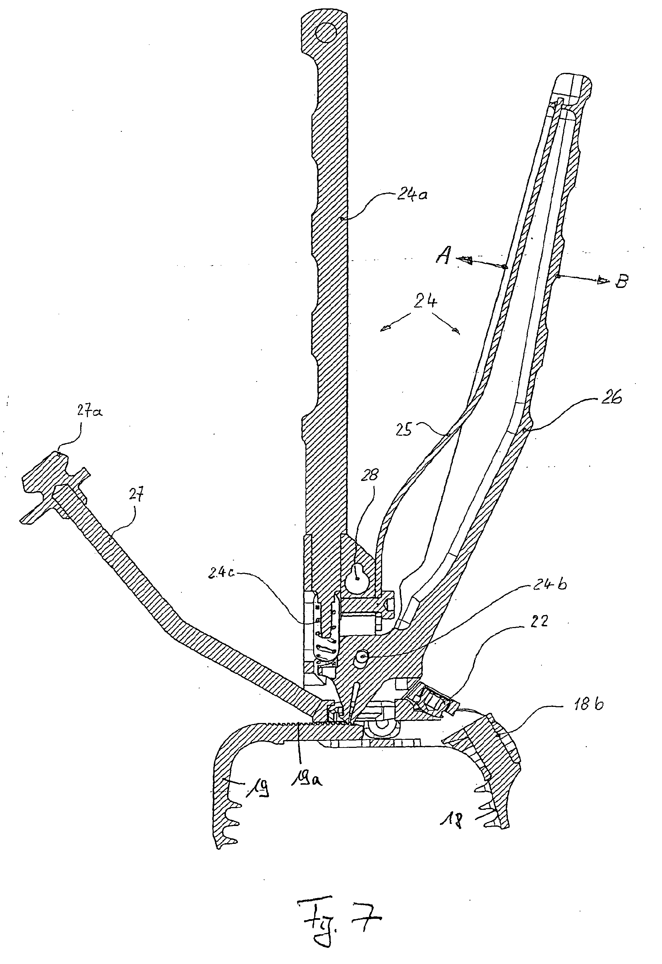

In den Figuren 6a bis 7b ist ein zweites Ausführungsbeispiel einer erfindungsgemäßen Knochenklammer dargestellt. Dieses Ausführungsbeispiel ist als Klammer für eine Kniegelenkimplantatoperation vorgesehen und beinhaltet nebst einer Adaptionsschnittstelle 17b, 17c für Aufsetzmittel oder Operationshilfsmittel wie Positioniermittel und Sägeführungen eine direkt in die erste Klammerhälfte 17 integrierte Trägerstange 27 mit Adaptionsstelle 27a zur Ankopplung eines Lokatorfeldes. Die direkte Integration der Trägerstange 27 ist so realisiert, dass deren feste Verbindung mit der ersten Klammerhälfte 17 während der ganzen Operation nicht stört. Wenn die fest integrierte Trägerstange 27 zeitweise eine Störung wäre, könnte sie alternativ über eine zweite auf der ersten Knochenklammerhälfte 17 zu realisierende Adaptionsschnittstelle angekoppelt werden.

Unter dem Begriff "Adaptionsschnittstelle" werden Anschlußmittel verstanden, die angepaßt sind, um eine Verbindung der beiden Klammerteile 17, 19 mit einem separaten Stellorgan 24 bzw. Aufsetzhilfsmittel zu ermöglichen. Diese Anschlußmittel können beispielsweise als Gewindebohrungen oder als andere Verbindungselemente ausgeführt sein, vorausgesetzt, die Übertragung einer zum Befestigen der Klammer am Knochen ausreichenden Stellkraft wird ermöglicht.

Die zweite Knochenklammerhälfte 19 (Fig. 6a) ist ähnlich dem Klammerteil 2 im ersten Ausführungsbeispiel ausgebildet. Die erste Knochenklammerhälfte 17 besitzt einen über Achszapfen 16 schwenkgelagerten Arretierbügel 15, dessen Zahnpartie 15a mittels Federelement 23, auf die Gegenverzahnung 19a gedrückt wird. In Fig. 6b ist der Arretierbügel 15 in einer ausgeschwenkten Stellung dargestellt, wodurch sich die zweite Klammerhälfte 19 in der Innenführung der ersten Klammerhälfte 17 in Führungsrichtung frei bewegen lässt. Wie diese ausgeschwenkte Stellung gegen die Federkraft erreicht wird, ist der später folgenden Funktionsbeschreibung des Aufsetzhilfsmittels 24 bzw. allgemein des Stellorgans zu entnehmen.

Als Teil der Innenführung der ersten Knochenklammerhälfte 17 dient ein Auflagesteg 17a im Zusammenwirken mit weiteren Innenführungskonturen der ersten Klammerhälfte 17. Um größeren anatomischen Unterschieden der Knochenformen gerecht zu werden, ist das Armende 18 mit seinen Verankerungselementen 18a als mittels Zapfenlager 18b drehgeführtes Teil in einer zum Zapfenlager korrespondierenden Lagerbohrung des ersten Klammerteils 17 ausgeführt.

Die obere Abschlusspartie des ersten Klammerteils 17 und insbesondere die Formelemente 17b, 17c, dienen als Adaptionselemente für das Aufsetzen sowohl des Aufsetzhilfsmittels 24 als auch zur Aufnahme von Operationshilfsmitteln, für deren Einsatz dieses Ausführungsbeispiel insbesondere konzipiert ist. Der eigentlichen Fixierung von Aufsetzhilfsmittel 24 oder Operationshilfsmitteln auf der Klammerhälfte 17 dient ein Spannbügel 20, welcher mittels Zapfenlager 21 am ersten Klammerteil 17 gelagert ist und mittels Druckschraube 22 so aus dem Klammerteil 17 in eine Schwenkposition gebracht werden kann, dass sich die Adaptionspartien von aufzusetzenden Hilfsmitteln lagefixiert an einer bei wiederholtem Aufsetzen genau positionsgleichen Stelle am ersten Klammerteil 17 verankern lassen.

Das Aufsetzhilfsmittel 24 umfaßt ein Hauptteil 24a mit korrespondierender Adaptionsschnittstelle zur oberen Abschlusspartie der ersten Klammerhälfte 17 bzw. deren Adaptionsschnittstellen 17b, 17c und besitzt im oberen Bereich eine erste Griffpartie 24a und im Mittelteil eine Lagerstelle 24b, welche im Zusammenwirken mit einer Ausnehmung als Führungspartie das Griffteil 26 hält.

Das Griffteil 26 ist mittels Federelement 25 in einer mittleren Drehsollposition um die Lagerstelle 24b gehalten und durch Schwerkraft oder ein Federelement 24c innerhalb seiner um die Lagerstelle 24b angeordneten Langlochbohrung in Richtung gegen die zweite Klammerhälfte 19 aufliegend angeordnet. In dieser Auflagepartie besitzt das Griffteil 26 Gegenzahnelemente 26a, welche im Eingriff mit der Zahnreihe 19a bei Betätigung des Griffteils 26 in Pfeilrichtung A einen Schließvorgang der Klammer um eine oder mehrere Zahnpositionen zwischen der zweiten Klammerhälfte 19 und dem Arretierbügel 15 bewirken. Beim Loslassen des Griffteils 26 hält der Arretierbügel 15 die zweite Klammerhälfte 19 in seiner nun bestehenden Arretierposition fest und das Federelement 25 bringt das Griffteil 26 wieder in seine Mittelposition zurück, wobei die Zahnrücken der Zähne 26a über die Zahnrücken der Zähne 19a gleiten. Dieser Vorgang kann so oft wiederholt werden, bis die Klammer mit genügender Spannkraft auf dem Knochen befestigt ist.

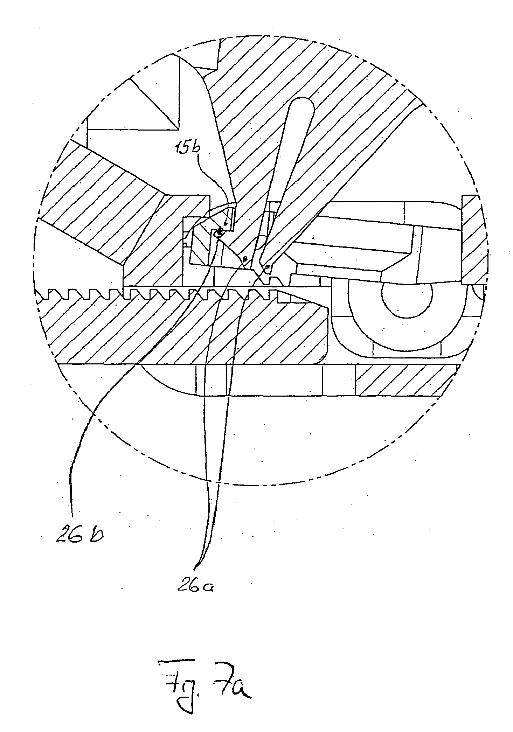

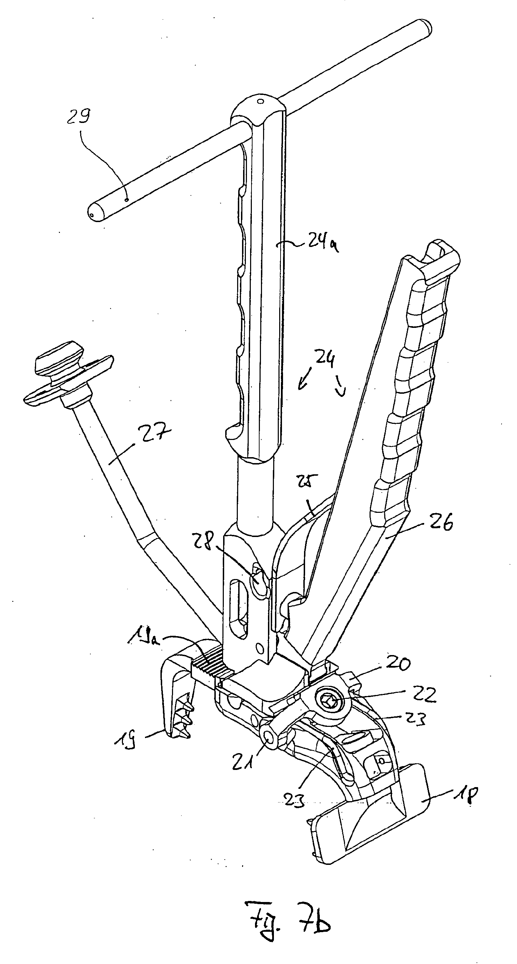

Zur Sicherheit gegen Überbelastung der Klammer könnte alternativ an geeigneter Stelle, wie z. B. in der Armpartie des Griffteils 26, eine Überlastsicherung wie beispielsweise eine Rutschkupplung vorgesehen sein. Sollte aus irgend einem Grund die Klammer wieder gelöst werden, solange das Aufsetzhilfsmittel 24 noch auf der Klammerhälfte 17 fixiert ist, kann mittels Betätigung des Griffteils 26 in die Bewegungsrichtung B eine Lösefunktion eingeleitet werden, wodurch der Haken 26b in eine Hinterschneidung 15b des Arretierbügels 15 greift, den Arretierbügel 15 aus dem Zahneingriff 15a, 19a aushebt und somit die Klammerspannung wieder löst (Fig. 7a).

Ohne aufgesetztes Aufsetzhilfsmittel 24 kann durch eine Seitenöffnung in der ersten Klammerhälfte 17 mittels eines Werkzeuges eine Kippbewegung durchgeführt werden, welche den Arretierbügel 15 gegen die Federkraft der Feder 23 wegkippt (analog der in Fig. 6b dargestellten Art) und so die Klammerarretierung löst.

Bei den nach dieser Ausführungsart zum Einsatz gelangenden Operationshilfsmitteln ist die am tiefsten liegende Partie der Adaptionsschnittstelle gegenüber der Schnittstelle des Aufsetzhilfsmittels 24 noch etwas tiefer liegend angeordnet und befindet sich unmittelbar über dem Arretierbügel 15 in seiner Arretierstellung, so dass kein Platz für ein Wegkippen des Arretierbügels 15 besteht. Somit besteht mit adaptiertem Operationshilfsmittel ein Schutz vor irrtümlichem Lösen der Klammerarretierung.

Das im Anwendungsfall bei dem zweiten Ausführungsbeispiel vorgesehene Operationshilfsmittel besitzt zwar mehrfach Feinpositioniereinrichtungen. Trotzdem ist es sinnvoll, dass zum Zweck einer gewissen Grobvorpositionierung an der Klammer oder insbesondere am Aufsetzhilfsmittel 24 Einrichtungen existieren, mit deren Hilfe der Chirurg die Klammerpositionierung auf dem Knochen näher an eine Idealposition bringen kann.

So ist hier ein länglich ausgebildetes Teil 29 vorgesehen, das nebst Grifffunktion auch eine Ziellinienfunktion dergestalt ausübt, indem die Längsachse von Teil 29 in etwa parallel zur Beinbelastungsachse auszurichten ist (Fig. 7b).

Im weiteren ist eine Bajonettöffnung 28 vorgesehen zur Ankoppelung einer Grobdistanzlehre. Damit kann die Entfernung zwischen dem Notchpunkt des Knies und der zu platzierenden Klammer abgeleitet werden (Fig. 7).

### Bezugszeichen

- 1, 2: Klammerteile/Klammerhälften
- 3: Innenführung
- 3': Außenführung
- 4, 4': Zähne
- 5, 5': Klammerarme
- 7, 7a: Adaptionsteil
- 7', 7": Anschlußmittel
- 8: Klammerpartie
- 8': Öffnungsspur
- 9: Aufsetzhilfsmittel/Stellorgan
- 9a, 9b: Armteile
- 9c: Winkelausleger
- 9d: Schnappbügel
- 10, 10a: Schrauben
- 12: Verbindungselement
- 13: Tragstange
- 13a: Endpartie
- 14: Markerfeld
- 15: Arretierbügel
- 15a: Zahnpartie
- 15b: Hinterschneidung
- 16: Achszapfen
- 17: Klammerteile/Klammerhälften
- 17a: Auflagesteg
- 17b, 17c: Adaptionsschnittstellen
- 18: Armende
- 18a: Verankerungselemente
- 18b: Zapfenlager
- 19: Klammerteile/Klammerhälften
- 19a: Gegenverzahnung/Zahnreihe
- 20: Spannbügel
- 21: Zapfenlager
- 22: Druckschraube
- 23: Federelement
- 24: Aufsatzhilfsmittel/Stellorgan
- 24a: Griffpartie
- 24b: Lagerstelle
- 24c, 25: Federelement
- 26: Griffteil
- 26a: Gegenzahnelemente
- 26b: Haken
- 27: Trägerstange
- 28: Bajonettöffnung
- 30: Arretierelement

## Patentansprüche

1. Klammer zur Befestigung chirurgischer Operationshilfsmittel (14) mit mindestens zwei relativ zueinander beweglich angeordneten Klammerteilen (1, 2; 17, 19),
**gekennzeichnet durch**
ein Arretierelement (30) zur Festlegung der Klammerteile (1, 2; 17, 19) in mindestens einer Sperrstellung, wobei die Klammerteile (1, 2; 17, 19) Anschlussmittel (7', 7") aufweisen derart, dass die Klammerteile (1, 2; 17, 19) mit einem separaten Stellorgan (9; 24) zur Übertragung einer Stellkraft verbindbar sind, wobei die Klammerteile (1, 2; 17, 19) mittels des Stellorgans (9; 24) in die Sperrstellung und/oder in eine Freigabestellung bringbar sind.

2. Klammer nach Anspruch 1,
**dadurch gekennzeichnet, dass**
die Klammer mittels des Stellorgans (9; 24) setzbar, spannbar und wieder lösbar ist, wobei dazu das Arretierelement (30) aus der Sperrstellung in eine Freigabestellung und umgekehrt bewegbar angeordnet ist.

3. Klammer nach Anspruch 1 oder 2,
**dadurch gekennzeichnet, dass**
die Anschlussmittel (7', 7") für das separate Stellorgan (9; 24) zur Befestigung der Operationshilfsmittel (14) angepasst sind.

4. Klammer nach einem der Ansprüche 1 bis 3,
**dadurch gekennzeichnet, dass**
die Klammerteile (1, 2; 17, 19) durch eine Linearführung (3, 3') oder durch ein Drehgelenk verbunden sind.

5. Klammer nach einem der Ansprüche 1 bis 4,
**dadurch gekennzeichnet, dass**
das Arretierelement (30) Rastmittel (4, 4'), insbesondere Zähne, aufweist, die den Klammerteilen (1, 2; 17, 19) zugeordnet und in der Sperrstellung verrastbar sind.

6. Klammer nach einem der Ansprüche 1 bis 5,
**dadurch gekennzeichnet, dass**
ein Löseelement (8, 8') vorgesehen ist, dass mit dem Arretierelement (30) zum Lösen der Klammerteile (1, 2; 17, 19) aus der Sperrstellung zusammenwirkt.

7. Klammer nach Anspruch 6,
**dadurch gekennzeichnet, dass**
das Arretierelement (30) mit einer Rückstellkraft beaufschlagt ist, die das Arretierelement (30) in die Sperrstellung bewegt und/oder in der Sperrstellung hält.

8. Stellorgan (9; 24), insbesondere Zange, zur lösbaren Verbindung mit einer Klammer gemäß einem der Ansprüche 1 bis 7,
wobei das Stellorgan (9) Anschlussmittel (10, 10a) aufweist, die mit den Anschlussmitteln (7', 7") der Klammer verbindbar sind.

9. Stellorgan (9; 24) nach Anspruch 8,
**dadurch gekennzeichnet, dass**
das Stellorgan (9; 24) Abstands- und/oder Ausrichtmittel umfasst.

10. Anordnung zur Befestigung chirurgischer Operationsmittel umfassend eine Klammer gemäß einem der Ansprüche 1 bis 7 und ein mit der Klammer lösbar verbundenes Stellorgan (9; 24) gemäß Anspruch 8 oder 9.
